# EUROPEAN PATENT SPECIFICATION

(11) **EP 2 183 247 B1**
(45) Date of publication and mention of the grant of the patent: **16.05.2012**
(21) Application number: 08826701.8
(22) Date of filing: 29.07.2008
(51) Int. Cl.: C07D 413/14, A61K 31/5377, A61P 19/04, A61P 19/10, A61P 25/16, A61P 25/18, A61P 25/28, A61P 29/00, A61P 3/10

(54) **NEW CRYSTALLINE FORMS OF 2 -HYDROXY- 3- [5- (MORPHOLIN- 4- YLMETHYL) PYRIDIN-2-YL]1H- INDOLE- 5 -CARBONITRILE CITRATE**
NEUE KRISTALLINE FORMEN VON 2-HYDROXY-3-[5-(MORPHOLIN-4-YLMETHYL)PYRIDIN-2-YL]1H-INDOL-5-CARBONITRILCITRAT
NOUVELLES FORMES CRISTALLINES DE CITRATE DE 2-HYDROXY-3-[5-(MORPHOLIN-4- YLMÉTHYL)PYRIDIN-2-YL]1H-INDOLE-5-CARBONITRILE

(30) Priority: 30.07.2007 US 952634 P
(43) Date of publication of application: 12.05.2010
(73) Proprietor: AstraZeneca AB, 151 85 Södertälje (SE)
(72) Inventor: ERIKSON, Anders, S-151 85 Södertälje (SE); PROFIR, Veronica, S-151 85 Södertälje (SE); SEBHATU, Tesfai, S-151 85 Södertälje (SE); TJERNELD, Erica, S-151 85 Södertälje (SE)
(86) International application number: PCT/SE2008/050895
(87) International publication number: WO 2009/017452

(56) References cited:
- WO-A1-03/048162
- WO-A1-03/082853
- WO-A1-2007/089191
- WO-A1-2008/130312

## Description

### FIELD OF THE INVENTION

The present invention relates to new crystalline forms of 2-hydroxy-3-[5-(morpholin-4-ylmethyl)pyridin-2-yl]1*H*-indole-5-carbonitrile citrate, a Form D and a Form E, respectively, processes for their preparations, pharmaceutical formulations containing said compounds and to the use of said active compounds in therapy.

### BACKGROUND OF THE INVENTION

2-Hydroxy-3-[5-(morpholin-4-ylmethyl)pyridin-2-yl]1*H*-indole-5-carbonitrile as a free base and the hydrochloride salt thereof are described in WO 03/082853. This compound is useful because it possess pharmacological activity by showing inhibiting effect on GSK3 (WO 03/082853). This compound could be used to treat Alzheimer disease, dementias, chronic and acute neurodegenerative diseases, bipolar disorders, schizophrenia, diabetes, hair loss, bone-related disorders and all the listed disorders described in WO 03/082853, which hereby are incorporated into this specification by reference.

2-Hydroxy-3-[5-(morpholin-4-ylmethyl)pyridin-2-yl]1*H*-indole-5-carbonitrile citrate and its crystalline form, Form A, are described in WO 07/089191.

Glycogen synthase kinase 3 (GSK3) is a serine / threonine protein kinase composed of two isoforms (α and β), which are encoded by distinct genes but are highly homologous within the catalytic domain. GSK3 is highly expressed in the central and peripheral nervous system. GSK3 phosphorylates several substrates including tau, ß-catenin, glycogen synthase, pyruvate dehydrogenase and elongation initiation factor 2b (eIF2b). Insulin and growth factors activate protein kinase B, which phosphorylates GSK3 on serine 9 residue and inactivates it.

### Alzheimer's Disease (AD) dementias, and taupathies.

AD is characterized by cognitive decline, cholinergic dysfunction and neuronal death, neurofibrillary tangles and senile plaques consisting of amyloid-β deposits. The sequence of these events in AD is unclear, but is believed to be related. Glycogen synthase kinase 3β (GSK3β) or Tau phosphorylating kinase selectively phosphorylates the microtubule associated protein Tau in neurons at sites that are hyperphosphorylated in AD brains. Hyperphosphorylated tau has lower affinity for microtubules and accumulates as paired helical filaments, which are the main components that constitute neurofibrillary tangles and neuropil threads in AD brains. This results in depolymerization of microtubules, which leads to dying back of axons and neuritic dystrophy. Neurofibrillary tangles are consistently found in diseases such as AD, amyotrophic lateral sclerosis, parkinsonism-dementia of Gaum, corticobasal degeneration, dementia pugilistica and head trauma, Down's syndrome, postencephalatic parkinsonism, progressive supranuclear palsy, Niemann-Pick's Disease and Pick's Disease. Addition of amyloid-β to primary hippocampal cultures results in hyperphosphorylation of tau and a paired helical filaments-like state via induction of GSK3β activity, followed by disruption of axonal transport and neuronal death (Imahori and Uchida, J. Biochem 121:179-188, 1997). GSK3β preferentially labels neurofibrillary tangles and has been shown to be active in pre-tangle neurons in AD brains. GSK3 protein levels are also increased by 50% in brain tissue from AD patients. Furthermore, GSK3β phosphorylates pyruvate dehydrogenase, a key enzyme in the glycolytic pathway and prevents the conversion of pyruvate to acetyl-Co-A (Hoshi et al., PNAS 93:2719-2723, 1996). Acetyl-Co-A is critical for the synthesis of acetylcholine, a neurotransmitter with cognitive functions. Accumulation of amyloid-β is an early event in AD. GSK Tg mice show increased levels of amyloid-β in brain. Also, PDAPP mice fed with Lithium show decreased amyloid-β levels in hippocampus and decreased amyloid plaque area (Su et al., Biochemistry 2004, 43:6899-6908). Thus, GSK3β inhibition may have beneficial effects in progression as well as the cognitive deficits associated with Alzheimer's disease and other above-referred to diseases.

### Chronic and Acute Neurodegenerative Diseases

Growth factor mediated activation of the PI3K /Akt pathway has been shown to play a key role in neuronal survival. The activation of this pathway results in GSK3β inhibition. Recent studies (Bhat et. al., PNAS 97:11074-11079 (2000)) indicate that GSK3β activity is increased in cellular and animal models of neurodegeneration such as cerebral ischemia or after growth factor deprivation. For example, the active site phosphorylation was increased in neurons vulnerable to apoptosis, a type of cell death commonly thought to occur in chronic and acute degenerative diseases such as cognitive disorders, Alzheimer's Disease, Parkinson's Disease, amyotrophic lateral sclerosis, Huntington's Disease and HIV dementia and traumatic brain injury; and as in ischemic stroke. Lithium was neuroprotective in inhibiting apoptosis in cells and in the brain at doses that resulted in the inhibition of GSK3β. Thus GSK3β inhibitors could be useful in attenuating the course of neurodegenerative diseases.

### Bipolar Disorders (BD)

Bipolar Disorders are characterised by manic episodes and depressive episodes. Lithium has been used to treat BD based on its mood stabilising effects. The disadvantage of lithium is the narrow therapeutic window and the danger of overdosing that can lead to lithium intoxication. The discovery that lithium inhibits GSK3 at therapeutic concentrations has raised the possibility that this enzyme represents a key target of lithium's action in the brain (Stambolic et al., Curr. Biol. 6:1664-1668, 1996; Klein and Melton; PNAS 93:8455-8459, 1996; Gould et al., Neuropsychopharmacology, 1:32-8, 2004). GSK3 inhibitor has been shown to reduce immobilisation time in forced swim test, a model to assess depressive behavior (O'Brien et al., J Neurosci 2004, 24:66791-6798) GSK3 has been associated with a polymorphism found in bipolar II disorder (Szczepankiewicz et al., Neuropsychobiology. 2006;5 3(1):51-6). Inhibition of GSK3β may therefore be of therapeutic relevance in the treatment of BD as well as in AD patients that have affective disorders.

### Schizophrenia

Accumulating evidence implicates abnormal activity of GSK3 in mood disorders and schizophrenia. GSK3 is involved in signal transduction cascades of multiple cellular processes, particularly during neural development. Kozlovsky et al (Am J Psychiatry 2000 May; 157(5):831-3) found that GSK3β levels were 41% lower in the schizophrenic patients than in comparison subjects. This study indicates that schizophrenia involves neurodevelopmental pathology and that abnormal GSK3 regulation could play a role in schizophrenia. Furthermore, reduced β-catenin levels have been reported in patients exhibiting schizophrenia (Cotter et al., Neuroreport 9:1379-1383 (1998)). Atypical antipsychotics such as olanzapine, clozapine, quetiapine, and ziprasidone, inhibits GSK3 by increasing ser9 phosphorylation suggesting that antipsychotics may exert their beneficial effects via GSK3 inhibition (Li X et al., Int J Neuropsychopharmacol, 10(1):7-19 (2007).

### Diabetes

Insulin stimulates glycogen synthesis in skeletal muscles via the dephosphorylation and thus activation of glycogen synthase. Under resting conditions, GSK3 phosphorylates and inactivates glycogen synthase via dephosphorylation. GSK3 is also over-expressed in muscles from Type II diabetic patients (Nikoulina et al., Diabetes 2000 Feb; 49(2):263-71). Inhibition of GSK3 increases the activity of glycogen synthase thereby decreasing glucose levels by its conversion to glycogen. In animal models of diabetes, GSK3 inhibitors lowered plasma glucose levels up to 50 % (Cline et al., Diabetes, 2002, 51:2903-2910; Ring et at., Diabetes 2003, 52:588-595). GSK3 inhibition may therefore be of therapeutic relevance in the treatment of Type I and Type II diabetes and diabetic neuropathy.

### Alopecia

GSK3 phosphorylates and degrades β-catenin. β-catenin is an effector of the pathway for keratonin synthesis. β-catenin stabilisation may be lead to increase hair development. Mice expressing a stabilised β-catenin by mutation of sites phosphorylated by GSK3 undergo a process resembling de novo hair morphogenesis (Gat et al., Cell 1998 Nov 25;95 (5):605-14)). The new follicles formed sebaceous glands and dermal papilla, normally established only in embryogenesis. Thus GSK3 inhibition may offer treatment for baldness.

### Bone-related disorders and conditions

GSK3 inhibitors could be used for treatment of bone-related disorders or other conditions, which involves a need for new and increased bone formation. Remodeling of the skeleton is a continuous process, controlled by systemic hormones such as parathyroid hormone (PTH), local factors (e.g. prostaglandin E₂), cytokines and other biologically active substances. Two cell types are of key importance: osteoblasts (responsible for bone formation) and osteoclasts (responsible for bone resorption). Via the RANK, RANK ligand and osteoprotegerin regulatory system these two cell types interact to maintain normal bone turnover (Bell NH, Current Drug Targets - Immune, Endocrine & Metabolic Disorders, 2001, 1:93-102).

Osteoporosis is a skeletal disorder in which low bone mass and deterioration of bone microarchitecture lead to increased bone fragility and fracture risk. To treat osteoporosis, the two main strategies are to either inhibit bone resorption or to stimulate bone formation. The majority of drugs currently on the market for the treatment of osteoporosis act to increase bone mass by inhibiting osteoclastic bone resorption. It is recognized that a drug with the capacity to increase bone formation would be of great value in the treatment of osteoporosis as well as having the potential to enhance fracture healing in patients.

The use of GSK3 inhibitors in primary and secondary osteoporosis, where primary osteoporosis includes postmenaupausal osteoporosis and senile osteoporosis in both men and women, and secondary osteoporosis includes cortison induced osteoporosis, as well as any other type of induced secondary osteoporosis. In addition to this, GSK3 inhibitors may also be used in treatments of myeloma. The GSK3 inhibitors may be administered locally or systemically, in different formulation regimes, to treat these conditions.

### Inflammatory disease

The discovery that GSK3 inhibitors provide anti-inflammatory effects has raised the possibility of using GSK3 inhibitors for therapeutic intervention in inflammatory diseases. (Martin et al., Nat Immunol 2005, 6:777-784; rev. in Jope et al., Neurochem Res 2006, Aug 30). Inflammation is a common feature of a broad range of conditions including Alzheimer's Disease and mood disorders.

### DETAILED DESCRIPTION OF THE INVENTION

The object of the present invention is to provide the compound 2-hydroxy-3-[5-(morpholin-4-ylmethyl)pyridin-2-yl]1*H*-indole-5-carbonitrile citrate in its crystalline forms, Form D and Form E, respectively. Said compounds having a selective inhibiting effect at GSK3 and a low hygroscopicity which making it suitable to be formulated into pharmaceutical formulations.

The substantially crystalline citrate salt of the compound 2-hydroxy-3-[5-(morpholin-4-ylmethyl)pyridin-2-yl]1*H*-indole-5-carbonitrile according to the present invention have been found to show an improved chemical stability over the hydrochloride salt of 2-hydroxy-3-[5-(morpholin-4-ylmethyl)pyridin-2-yl]1*H*-indole-5-carbonitrile prepared as described in WO 03/082853 which makes them particularly suitable to be formulated into pharmaceutical formulations.

The crystalline Form D of 2-hydroxy-3-[5-(morpholin-4-ylmethyl)pyridin-2-yl]1*H*-indole-5-carbonitrile citrate exists as an anhydrate.

The crystalline Form E of 2-hydroxy-3-[5-(morpholin-4-ylmethyl)pyridin-2-yl]1*H*-indole-5-carbonitrile citrate exists as a monohydrate and as such it has shown a very low hygroscopicity.

The crystalline anhydrate Form D may be transformed into the hydrated crystalline Form E and vice versa, therefore mixtures of these two forms Form D and Form E may exist.

In the formulation of pharmaceutical formulations, it is important for the pharmaceutically acceptable compound (the active drug compound) to be in a form in which it can be conveniently handled and processed. This is of importance, not only from the point of view of obtaining commercially viable manufacturing process, but also from the point of view of subsequent manufacture of pharmaceutical formulations comprising the active drug compound.

Chemical stability, solid state stability, and "shelf-life" of the active ingredients are also very important factors. The drug compound and formulations containing it should be capable of being effectively stored over appreciable periods of time, without exhibiting a significant change in physico-chemical characteristics of the active component, e.g. its chemical composition, density, hygroscopicity and solubility.

The term "chemical stability" means that the compound can be stored in an isolated form, or in the form of a formulation in which it is provided in admixture with pharmaceutically acceptable carriers, diluents or adjuvants (*e.g*., in an oral dosage form, such as tablet, capsule, etc.), under normal storage conditions, with little or no chemical degradation or decomposition.

Thus, in the manufacture of commercially viable and pharmaceutically acceptable drug formulations it is important, wherever possible, to provide the drug compound in a substantially crystalline and stable form.

As used herein, the term "substantially crystalline" means at least about 50% crystalline and ranging up to 100% crystalline. The present invention provides 2-hydroxy-3-[5-(morpholin-4-ylmethyl)pyridin-2-yl]1*H*-indole-5-carbonitrile citrate that is at least about 50% crystalline, at least about 60% crystalline, at least about 70% crystalline, at least about 80% crystalline, at least about 90% crystalline, at least about 95% crystalline, at least about 98% crystalline, or about 100% crystalline in form.

### PHARMACEUTICAL FORMULATIONS

According to one aspect of the present invention there is provided a pharmaceutical formulation comprising the compound 2-hydroxy-3-[5-(morpholin-4-ylmethyl)pyridin-2-yl]1*H*-indole-5-carbonitrile citrate in its crystalline forms, Form D and Form E, respectively, for use in the prevention and/or treatment of conditions associated with glycogen synthase kinase-3.

The formulation may be in a form suitable for oral administration, for example as a tablet, for parenteral injection as a sterile solution or suspension, for local administration in a body cavity or in a bone cavity, for example as a sterile injection solution or suspension.

In general the above formulation may be prepared in a conventional manner using pharmaceutically carriers or diluents. Suitable daily doses of the salt of the compound of the present invention in the treatment of a mammal, including man, are approximately 0.01 to 250 mg/kg bodyweight at peroral administration and about 0.001 to 250 mg/kg bodyweight at parenteral administration. The typical daily dose of the active ingredients varies within a wide range and will depend on various factors such as the relevant indication, the route of administration, the age, weight and sex of the patient and may be determined by a physician.

For the veterinary use the amounts of different components, the dosage form and the dose of the medicament may vary and will depend on various factors as for example the individual requirement of the animal treated.

The compound of the present invention, can be used on its own but will usually be administered in the form of a pharmaceutical formulation in which the compound salt (active ingredient) is in association with a pharmaceutically acceptable diluent or carrier. Dependent on the mode of administration, the pharmaceutical formulation may comprise from 0.05 to 99 % w/w (per cent by weight), for example from 0.10 to 50 % w/w, of active ingredient, all percentages by weight being based on total composition.

A diluent or inert carrier includes water, aqueous poly(ethylene glycol), magnesium carbonate, magnesium stearate, talc, a sugar (such as lactose), pectin, dextrin, starch, tragacanth, microcrystalline cellulose, methyl cellulose, sodium carboxymethyl cellulose or cocoa butter.

A formulation of the invention can be in tablet or injectable form. The tablet may additionally comprise a disintegrant and/or may be coated (for example with an enteric coating or coated with a coating agent such as hydroxypropyl methylcellulose).

The invention further provides a process for the preparation of a pharmaceutical formulation of the invention which comprises mixing of the compound, as hereinbefore defined, with a pharmaceutically acceptable diluent or inert carrier.

An example of a pharmaceutical formulation of the invention is an injectable solution containing a compound of the invention, as hereinbefore defined, and sterile water, and, if necessary, either sodium hydroxide or hydrochloric acid to bring the pH of the final formulation to about pH 5, and optionally a surfactant to aid dissolution.

An example of a suitable formulation is a liquid solution comprising 2-hydroxy-3-[5-(morpholin-4-ylmethyl)pyridin-2-yl]1*H*-indole-5-carbonitrile citrate, as hereinbefore defined, 5.0% mg/mL dissolved in pure water to 100%.

### MEDICAL USES

It has been found that the new crystalline forms Form D and Form E, respectively, of 2-hydroxy-3-[5-(morpholin-4-ylmethyl)pyridin-2-yl]1*H*-indole-5-carbonitrile citrate defined in the present invention, are well suited for inhibiting glycogen synthase kinase-3 (GSK3). Accordingly, said compound of the present invention is expected to be useful in the prevention and/or treatment of conditions associated with glycogen synthase kinase-3 activity, i.e. the compounds may be used to produce an inhibitory effect of GSK3 in mammals, including man, in need of such prevention and/or treatment.

GSK3 is highly expressed in the central and peripheral nervous system and in other tissues. Thus, it is expected that compound of the invention is well suited for the prevention and/or treatment of conditions associated with glycogen synthase kinase-3 in the central and peripheral nervous system. In particular, the compound of the invention is expected to be suitable for prevention and/or treatment of conditions associated with cognitive disorders and predemented states, especially dementia, Alzheimer's Disease (AD), Cognitive Deficit in Schizophrenia (CDS), Mild Cognitive Impairment (MCI), Age-Associated Memory Impairment (AAMI), Age-Related Cognitive Decline (ARCD) and Cognitive Impairement No Dementia (CIND), diseases associated with neurofibrillar tangle pathologies, Frontotemporal dementia (FTD), Frontotemporal dementia Parkinson's Type (FTDP), progressive supranuclear palsy (PSP), Pick's Disease, Niemann-Pick's Disease, corticobasal degeneration (CBD), traumatic brain injury (TBI) and dementia pugilistica.

One embodiment of the invention relates to the prevention and/or treatment of Alzheimer's Disease, especially the use in the delay of the disease progression of Alzheimer's Disease.

Other conditions are selected from the group consisting of Down's syndrome, vascular dementia, Parkinson's Disease (PD), postencephelatic parkinsonism, dementia with Lewy bodies, HIV dementia, Huntington's Disease, amyotrophic lateral sclerosis (ALS), motor neuron diseases (MND, Creuztfeld-Jacob's disease and prion diseases.

Other conditions are selected from the group consisting of attention deficit disorder (ADD), attention deficit hyperactivity disorder (ADHD) and affective disorders, wherein the affective disorders are Bipolar Disorder including acute mania, bipolar depression, bipolar maintenance, major depressive disorders (MDD) including depression, major depression, mood stabilization, schizoaffective disorders including schizophrenia, and dysthymia.

Other conditions are selected from the group consisting of Type I diabetes, Type II diabetes, diabetic neuropathy, alopecia and inflammatory diseases.

One embodiment of the invention is directed to the use of the compounds, as hereinbefore defined, in the prevention and/or treatment of bone-related disorders in mammals.

Another aspect of the invention is directed to the use of the compounds, as hereinbefore defined, in the prevention and/or treatment of to treat osteoporosis in mammals.

One aspect of the invention is directed to the use of the compounds, as hereinbefore defined, to promote and/or increase bone formation in mammals.

One aspect of the invention is directed to the use of the compounds, as hereinbefore defined, to increase bone mineral density in mammals.

Another aspect of the invention is directed to the use of the compounds, as hereinbefore defined to reduce the rate of fracture and/or increase the rate of fracture healing in mammals.

Another aspect of the invention is directed to the use of the compounds, as hereinbefore defined, to increase cancellous bone formation and/or new bone formation in mammals.

The dose required for the therapeutic or preventive treatment of a particular disease or a particular condition will necessarily be varied depending on the host treated, the route of administration and the severity of the illness or injury being treated.

In the context of the present specification, the term "therapy" also includes "prevention" unless there are specific indications to the contrary. The terms "therapeutic" and "therapeutically" should be construed accordingly.

The invention also provides for a method of treatment and/or prevention of conditions associated with glycogen synthase kinase-3 comprising administrering to a mammal, including man or animal in need of such treatment and/or prevention a therapeutically effective amount of the compound 2-hydroxy-3-[5-(morpholin-4-ylmethyl)pyridin-2-yl]1*H*-indole-5-carbonitrile citrate in its crystalline forms, Form D and Form E, respectively.

Another aspect of the invention is wherein the compounds as defined herein, or a pharmaceutical composition or formulation comprising such a compound is administered concurrently, simultaneously, sequentially, separately or adjunct with another pharmaceutically active compound or compounds selected from the following:
(i) antidepressants such as agomelatine, amitriptyline, amoxapine, bupropion, citalopram, clomipramine, desipramine, doxepin duloxetine, elzasonan, escitalopram, fluvoxamine, fluoxetine, gepirone, imipramine, ipsapirone, maprotiline, nortriptyline, nefazodone, paroxetine, phenelzine, protriptyline, ramelteon, reboxetine, robalzotan, sertraline, sibutramine, thionisoxetine, tranylcypromaine, trazodone, trimipramine, venlafaxine and equivalents and pharmaceutically active isomer(s) and metabolite(s) thereof.
(ii) atypical antipsychotics including for example quetiapine and pharmaceutically active isomer(s) and metabolite(s) thereof.
(iii) antipsychotics including for example amisulpride, aripiprazole, asenapine, benzisoxidil, bifeprunox, carbamazepine, clozapine, chlorpromazine, debenzapine, divalproex, duloxetine, eszopiclone, haloperidol, iloperidone, lamotrigine, loxapine, mesoridazine, olanzapine, paliperidone, perlapine, perphenazine, phenothiazine, phenylbutylpiperidine, pimozide, prochlorperazine, risperidone, sertindole, sulpiride, suproclone, suriclone, thioridazine, trifluoperazine, trimetozine, valproate, valproic acid, zopiclone, zotepine, ziprasidone and equivalents and pharmaceutically active isomer(s) and metabolite(s) thereof.
(iv) anxiolytics including for example alnespirone, azapirones,benzodiazepines, barbiturates such as adinazolam, alprazolam, balezepam, bentazepam, bromazepam, brotizolam, buspirone, clonazepam, clorazepate, chlordiazepoxide, cyprazepam, diazepam, diphenhydramine, estazolam, fenobam, flunitrazepam, flurazepam, fosazepam, lorazepam, lormetazepam, meprobamate, midazolam, nitrazepam, oxazepam, prazepam, quazepam, reclazepam, tracazolate, trepipam, temazepam, triazolam, uldazepam, zolazepam and equivalents and pharmaceutically active isomer(s) and metabolite(s) thereof.
(v) anticonvulsants including for example carbamazepine, valproate, lamotrogine, gabapentin and equivalents and pharmaceutically active isomer(s) and metabolite(s) thereof.
(vi) Alzheimer's therapies including for example donepezil, memantine, tacrine and equivalents and pharmaceutically active isomer(s) and metabolite(s) thereof.
(vii) Parkinson's therapies including for example deprenyl, L-dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase and equivalents and pharmaceutically active isomer(s) and metabolite(s) thereof.
(viii) migraine therapies including for example almotriptan, amantadine, bromocriptine, butalbital, cabergoline, dichloralphenazone, eletriptan, frovatriptan, lisuride, naratriptan, pergolide, pramipexole, rizatriptan, ropinirole, sumatriptan, zolmitriptan, zomitriptan, and equivalents and pharmaceutically active isomer(s) and metabolite(s) thereof.
(ix) stroke therapies including for example abciximab, activase, NXY-059, citicoline, crobenetine, desmoteplase, repinotan, traxoprodil and equivalents and pharmaceutically active isomer(s) and metabolite(s) thereof.
(x) urinary incontinence therapies including for example darafenacin, falvoxate, oxybutynin, propiverine, robalzotan, solifenacin, tolterodine and and equivalents and pharmaceutically active isomer(s) and metabolite(s) thereof.
(xi) neuropathic pain therapies including for example gabapentin, lidoderm, pregablin and equivalents and pharmaceutically active isomer(s) and metabolite(s) thereof.
(xii) nociceptive pain therapies such as celecoxib, etoricoxib, lumiracoxib, rofecoxib, valdecoxib, diclofenac, loxoprofen, naproxen, paracetamol and equivalents and pharmaceutically active isomer(s) and metabolite(s) thereof.
(xiii) insomnia therapies including for example agomelatine, allobarbital, alonimid, amobarbital, benzoctamine, butabarbital, capuride, chloral, cloperidone, clorethate, dexclamol, ethchlorvynol, etomidate, glutethimide, halazepam, hydroxyzine, mecloqualone, melatonin, mephobarbital, methaqualone, midaflur, nisobamate, pentobarbital, phenobarbital, propofol, ramelteon, roletamide, triclofos, secobarbital, zaleplon, zolpidem and equivalents and pharmaceutically active isomer(s) and metabolite(s) thereof.
(xiv) mood stabilizers including for example carbamazepine, divalproex, gabapentin, lamotrigine, lithium, olanzapine, quetiapine, valproate, valproic acid, verapamil, and equivalents and pharmaceutically active isomer(s) and metabolite(s) thereof.

Such combination products employ the compound of this invention within the dosage range described herein and the other pharmaceutically active compound or compounds within approved dosage ranges and/or the dosage described in the publication reference.

### METHOD OF SALT FORMATION

The formation of the citrate salt of the compound, 2-hydroxy-3-[5-(morpholin-4-ylmethyl)pyridin-2-yl]1H-indole-5-carbonitrile, may be prepared by mixing 2-hydroxy-3-[5-(morpholin-4-ylmethyl)pyridin-2-yl]1H-indole-5-carbonitrile with citric acid in the presence of a solvent. The equivalent of citric acid may vary between 1 and 3 mole equivalents. The reaction may be performed in water or a mixture of water and an alcohol such as methanol, ethanol or propanol. The solvent, which is water or a mixture of water and ethanol are preferred. The ratio of water to alcohol may be in the range of about between 60 to 100%(v/v). The total volume of solvents used may vary between 1 (v/w) to 100 (v/w) volume parts per weight of starting material, preferably between 10 (v/w) and 45 (v/w) volumes parts per weight of starting material. The temperature of the reaction may be between -30 and +150°C, preferably between -5°C and +100°C.

Pure 2-hydroxy-3-[5-(morpholin-4-ylmethyl)pyridin-2-yl] 1H-indole-5-carbonitrile citrate may be obtained by crystallising with or without an additive in suitable solvents to obtain a crystalline solid having a purity of about 95%, preferably about 98%.

Objects of the present invention are the processes for preparation of the crystalline forms, Form D and Form E, respectively, of 2-hydroxy-3-[5-(morpholin-4-ylmethyl)pyridin-2-yl]1H-indole-5-carbonitrile citrate.

Thus, one of said object is a process for the preparation of a crystalline form of 2-hydroxy-3-[5-(morpholin-4-ylmethyl)pyridin-2-yl]1*H*-indole-5-carbonitrile citrate, the Form D, comprising the following steps:
a) suspending 2-hydroxy-3-[5-(morpholin-4-ylmethyl)pyhdin-2-yl]1*H*-indole-5-carbonitrile citrate of any form, or a mixture of any form in water;
b) heating the slurry to about 85°C until a solution is obtained;
c) crystallizing the obtained solution by cooling to about 45°C over about 30 min. and then further cooling down to about 5°C over about 20 hrs;
d) isolating the crystallized compound obtained, by filtering followed by washing with ethanol and drying under vacuum at about 50°C.

The other of said object is a process for the preparation of a crystalline form of 2-hydroxy-3-[5-(morpholin-4-ylmethyl)pyridin-2-yl]1*H*-indole-5-carbonitrile citrate, the Form E, comprising the following steps:
a) suspending 2-hydroxy-3-[5-(morpholin-4-ylmethyl)pyridin-2-yl]1*H*-indole-5-carbonitrile citrate of any form, or a mixture of any form in water;
b) heating the slurry to about 95°C until a solution is obtained;
c) cooling the obtained solution to about 75°C over about 90 min. and then adding ethanol in the ratio of about between 0% and 40% (v/v) to the water added;
d) crystallizing the obtained solution in step c) by further cooling to about 15°C over about 8 hrs and then stirring overnight;
e) isolating the crystallized compound obtained, by filtering followed by washing with acetone/water and pulled dry.

### ILLUSTRATIVE WORKING EXAMPLE

The following examples will describe, but not limit, the invention.

### Example 1

### Form D of 2-Hydroxy-3-[5-(morpholin-4-ylmethyl)pyridin-2-yl]1H-indole-5-carbonitrile citrate

To 2-hydroxy-3-[5-(morpholin-4-ylmethyl)pyridin-2-yl]1*H*-indole-5-carbonitrile citrate (4 g, 7.6 mmol) was added water (40 ml) and the slurry heated to 85°C until all was dissolved. Then the solution was cooled to 45°C over 30 min, followed by further cooling down to 5°C over 20 hrs. The crystals were filtered and washed with ethanol. Drying in a vacuum at 50°C gave 2-hydroxy-3-[5-(morpholin-4-ylmethyl)pyridin-2-yl]1*H*-indole-5-carbonitrile citrate (3.22g, 81%yield) with a purity of 98.9 area%.
¹H NMR (d6-DMSO, 400 MHz) δ 14.8 (br s, 1 H), 10.98 (s, 1H), 8.1 (s 1H), 7.89 (s, 1H); 7.76 (br d, J= 9Hz, 2H), 7.31 (dd, J= 8Hz, 1 H), 7.02 (d, J= 8Hz, 1H), 3.60 (t; J= 8Hz, 4H), 3.45 (m, 2H), 2.75 (ap d, 2H), 2.65 (ap d, 2H), 2.47 (s, 4H) ppm; ¹³C NMR (*d6*-DMSO, 400MHz) δ 174.9, 171.3, 168.7, 148.4, 142.1, 137.1, 136.4, 125.2, 124.1, 121.1, 118.8, 118.4, 108.8, 101.4, 84.6, 72.3, 65.7, 58.0, 52.5, 42.9 ppm; MS (ES) *m*/*z* [M⁺+1] 335.

The crystals were analysed by X-ray powder diffraction (XRPD). The diffractogram of Form D shows the following d-values given in Angstrom and relative intensities: 1**4.56(vs), 11.36(m), 9.11(m),** 7.95(m), 7.74(vw), 7.27(vw), 6.75(vw), 6.34(w), 6.10(vw), 5.84(vw), **5.57(m), 5.11(w),** 4.99(vw), 4.85(m), 4.69(s), 4.56(vw), 4.46(w), **4.29(w),** 4.10(vw), 4.03(m), 4.00(w), 3.94(m), 3.82(vw), 3.67(w), **3.52(s),** 3.37(w), 3.34(ww), 3.08(ww), 3.02(vw), 2.98(w), 2.88(vw), 2.82(vw), 2.78(vw), 2.75(vw), 2.72(vw), 2.71(vw), 2.62(w).

The most significant d-value given in Angstrom is: **14.56.**

The three significant d-values given in Angstrom are: **14.56, 11.36, 5.57.**

The significant d-values given in Angstrom are: **14.56,11.36,9.11, 5.57, 5.11, 4.29,3.52.**

The relative intensities (rel.int.) are less reliable and instead of numerical values the following definitions are used:

| Definitions used: | % Relativ Intensity |
|---|---|
| vs (very strong): | 100-60.0 |
| s (strong): | 60.0-25.0 |
| m (medium): | 25.0-10.0 |
| w (weak): | 10.0-6.0 |
| vw (very weak): | <6.0 |

The relative intensities were derived from diffractograms measured with variable slits

### Example 2

### Form E of 2-Hydroxy-3-[5-(morpholin-4-ylmethyl)pyridin-2-yl]1H-indole-5-carbonitrile citrate

To 2-hydroxy-3-[5-(morpholin-4-ylmethyl)pyridin-2-yl]1*H*-indole-5-carbonitrile citrate (11.7 g, 22.2 mmol) was added water (200 ml) and the slurry was heated to 95°C until all was dissolved. The solution was then cooled to 75°C over 90 min, then ethanol (39 ml) was added. The solution was further cooled to 15°C over 8 hrs then stirred at 15°C overnight. The crystals were filtered, washed with acetone/water (50/50, 170ml) then pulled dry which gave 2-hydroxy-3-[5-(morpholin4-ylmethyl)pyridin-2-yl]1*H*-indole-5-carbonitrile citrate (9.59g, 82% yield) with a purity of 99.5 area%.
¹H NMR (*d6*-DMSO, 400 MHz) and ¹³C NMR (*d6*-DMSO, 400MHz) show identical δ values as given in Example 1. MS (ES) *m*/*z* [M⁺+1] 335.

The crystals were analysed by X-ray powder diffraction (XRPD). The diffractogram of Form E shows the following d-values given in Angstrom and relative intensities: **15.51(vs), 11.90(w),** 9.30(vw), 7.98(vw), 7.74(m), 7.16(vw), 6.26(vw), **5.96(vw), 5.71(vw), 5.17(m),** 4.99(s), 4.85(w), 4.66(vw), 4.52(vw), 4.46(vw), 4.43(w), 4.23(vw), 4.10(vw), 4.01(m), 3.92(vw), 3.68(vw), **3.61(m),** 3.55(w), 3.35(vw), 3.17(vw), 3.13(vw), 3.04(vw), 2.91(vw), 2.88(vw), 2.84(w), 2.73(vw), 2.71(vw), 2.66(vw), 2.62(vw).

The most significant d-value given in Angstrom is: **15.51.**

The three significant d-values given in Angstrom are: **15.51, 11.90, 5.17.**

The significant d-values given in Angstrom are: **15.51, 11.90, 5.96, 5.71, 5.17, 3.61.**

The relative intensities (rel.int.) are less reliable and instead of numerical values the following definitions are used:

| Definitions used: | % Relativ Intensity |
|---|---|
| vs (very strong): | 100-65.0 |
| s (strong): | 65.0-30.0 |
| m (medium): | 30.0-14.5 |
| w (weak): | 14.5-7.0 |
| vw (very weak): | <7.0 |

The relative intensities were derived from diffractograms measured with variable slits.

Crystallinity of 2-hydroxy-3-[5-(morpholin-4-ylmethyl)pyridin-2-yl]1*H*-indole-5-carbonitrile citrate were analyzed using X-ray powder diffraction (XRPD) as described below:

The peaks, identified with d-values calculated from the Bragg formula and intensities, have been extracted from the diffractogram of polymorph D and E, respectively, of crystalline citrate salt. Only the main peaks, that are the most characteristic, significant, distinct and/or reproducible, have been tabulated, but additional peaks can be extracted, using conventional methods, from the diffractogram. The presence of these main peaks, reproducible and within the error limit, is for most circumstances sufficient to establish the presence of said different polymorphs of crystalline citrate salt. Merely loss of a peak does not mean that another crystalline form of the compound has been obtained.

X-ray powder diffraction analysis (XRPD) was performed on samples prepared according to standard methods, for example those described in Giacovazzo, C. et al (1995), Fundamentals of Crystallography, Oxford University Press; Jenkins, R. and Snyder, R. L. (1996), Introduction to X-Ray Powder Diffractometry, John Wiley & Sons, New York; Bunn, C. W. (1948), Chemical Crystallography, Clarendon Press, London; or Klug, H. P. & Alexander, L. E. (1974), X-ray Diffraction Procedures, John Wiley and Sons, New York. X-ray diffraction analyses were performed using a PANalytical X'Pert Pro MPD for 96 minutes from 1 to 60° 2θ. Calculation into d-values (distance values) was done and they may vary in the range ±2 on the last given decimal place.

Raman spectroscopy was used for in process control of the crystal modification, while the final identification was always performed by XRPD on wet or on mildly dried material.

A Raman Rxn3 Analyzer (Kaiser Optical Systems, Inc.) was used for the Raman spectroscopy measurements. Raman spectra were recorded using near infrared (NIR) excitation radiation at 785 nm and a multichannel charge-coupled device (CCD) detector with an spectral resolution of 5 cm⁻¹. Due to the instrument setup however, peak shifts can be detected at as low as 1.7 cm⁻¹. Spectra were recorded off-line on solid material. All spectra were scaled and baseline centred before evaluation by using the maximum peak between 1250-1350 cm⁻¹ and the minimum baseline between 1350-1400 cm⁻¹.

Figure 1 shows the Raman spectra of Form A (dotted line), Form D (dashed line) and Form E (solid line) between 580 and 1480 cm⁻¹. Two regions where differences are seen are marked by arrows.

Figure 2 shows the Raman spectra of Form A (dotted line), Form D (dashed line) and Form E (solid line) between 2170 and 2255 cm⁻¹. The peak for Form E is very close (ca. 2215 cm⁻¹) to the Form A peak (ca. 2217 cm⁻¹), but very different from the Form D peak (ca. 2209 cm⁻¹).

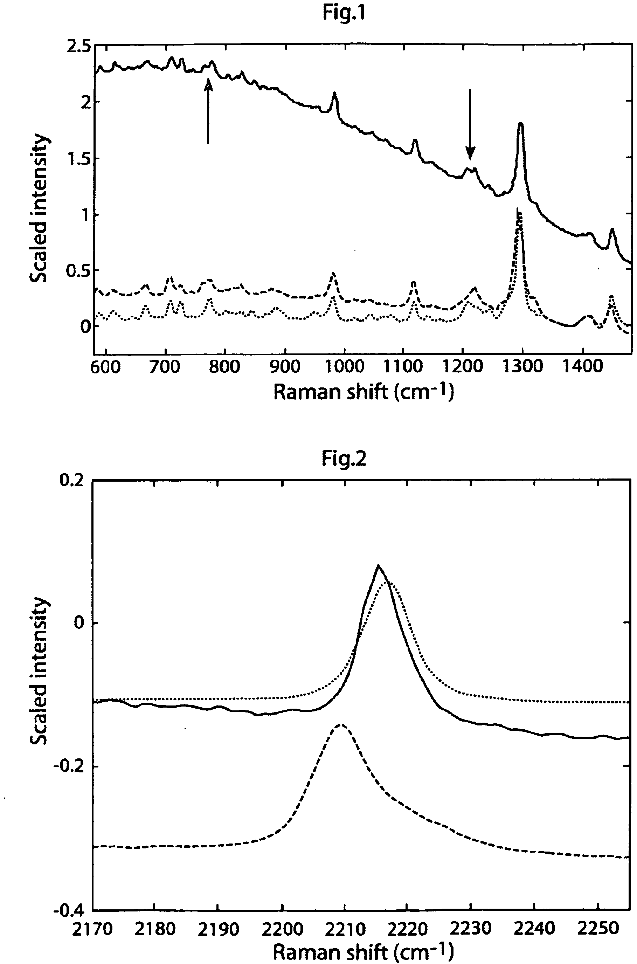

The Form E spectrum is then offset by 0.8 for visualisation purposes. The Raman spectrum recorded has several differences when compared to both Form A and Form D. The differences are seen both for peaks with medium intensity, such as the peaks at ca. 753-793 cm⁻¹ and 1186-1258 cm⁻¹ (marked by arrows in Figure 1) and also for the second largest peak in all spectra that has a different Raman shift for each form (see Figure 2): Form A ca. 2217 cm⁻¹, Form E ca. 2215 cm⁻¹ and Form D ca. 2209 cm⁻¹.

### PHARMACOLOGY

### Determination of ATP competition in Scintillation Proximity GSK3β Assay.

### GSK3β scintillation proximity assay.

The competition experiments were carried out in duplicate with 10 different concentrations of the inhibitor in clear-bottom microtiter plates (Wallac, Finland). A biotinylated peptide substrate, Biotin-Ala-Ala-Glu-Glu-Leu-Asp-Ser-Arg-Ala-Gly-Ser(PO₃H₂)-Pro-Gln-Leu (AstraZeneca, Lund), was added at a final concentration of 1 µM in an assay buffer containing 1 mU recombinant human GSK3β (Dundee University, UK), 12 mM morpholinepropanesulfonic acid (MOPS), pH 7.0, 0.3 mM EDTA, 0.01% β-mercaptorethanol, 0.004 % Brij 35 (a natural detergent), 0.5 % glycerol and 0.5 µg BSA/25 µl. The reaction was initiated by the addition of 0.04 µCi [γ-³³P]ATP (Amersham, UK) and unlabelled ATP at a final concentration of 1 µM and assay volume of 25 µl. After incubation for 20 minutes at room temperature, each reaction was terminated by the addition of 25 µl stop solution containing 5 mM EDTA, 50 µM ATP, 0.1 % Triton X-100 and 0.25 mg streptavidin coated Scintillation Proximity Assay (SPA) beads (Amersham, UK). After 6 hours the radioactivity was determined in a liquid scintillation counter (1450 MicroBeta Trilux, Wallac). The inhibition curves were analysed by non-linear regression using GraphPad Prism, USA. The Kₘ value of ATP for GSK3β, used to calculate the inhibition constants (Kᵢ) of the compound, was 20 µM.

The following abbreviations have been used:
- MOPS: Morpholinepropanesulfonic acid
- EDTA: Ethylenediaminetetraacetic acid
- BSA: Bovin Serum Albumin
- ATP: Adenosine Triphosphate
- SPA: Scintillation Proximity Assay
- GSK3: Glycogen synthase kinase 3

### Results

The GSK3β Kᵢ value for 2-hydroxy-3-[5-(morpholin-4-ylmethyl)pyridin-2-yl]1*H*-indole-5-carbonitrile citrate is 31 nM.

### HYGROSCOPICITY

### Dynamic vapour sorption analysis (DVS)

The studies were undertaken using Dynamic Vapour Sorption Appararatus (DVS, Surface Measurement Systems, London UK). The apparatus consists of Cahn micobalance housed inside a temperature-controled cabinet. All experiments were performed at 25 °C. The DVS was used to characterize the moisture uptake (w/w%) at different relative humidities (RH). Samples (5-10 mg) were weighed directly into the DSV sample cup and exposed to different relative humidities.

### Results

| Sample | | Relative humidities (RH) | |
|---|---|---|---|
| | 40% | 60% | 80% |
| HCl | 8.7 | 11.5 | 13.0 |
| Citrate Form A | 7.1 | 7.6 | 8.0 |
| Citrate Form D | 3.2 | 3.6 | 3.7 |
| Citrate Form E | 0.45 | 0.52 | 0.60 |

It is clear from the results above that the citrate salt of 2-hydroxy-3-[5-(morpholin-4-ylmethyl)pyridin-2-yl]1*H*-indole-5-carbonitrile shows a lower hygroscopicity than the hydrochloride salt thereof and is thus more suitable for preparing pharmaceutical formulations.

## Claims

1. A compound 2-hydroxy-3-[5-(morpholin-4-ylmethyl)pyridin-2-yl]1*H*-indole-5-carbonitrile citrate anhydrate in substantially crystalline form, which is a Form D **characterized by** the X-ray powder diffraction d-value **14.56** Å.

2. A compound 2-hydroxy-3-[5-(morpholin-4-ylmethyl)pyridin-2-yl]1*H*-indole-5-carbonitrile citrate anhydrate in substantially crystalline form, which is a Form D **characterized by** the X-ray powder diffraction d-values **14.56, 11.36, 5.57** Å.

3. A compound 2-hydroxy-3-[5-(morpholin-4-ylmethyl)pyridin-2-yl]1*H*-indole-5-carbonitrile citrate anhydrate in substantially crystalline form, which is a Form D **characterized by** the X-ray powder diffraction d-values and relative intensity **14.56, 11.36, 9.11, 5.57, 5.11, 4.29, 3.52** Å.

4. A process for the preparation of a substantially crystalline form of 2-hydroxy-3-[5-(morpholin-4-ylmethyl)pyridin-2-yl]1*H*-indole-5-carbonitrile citrate anhydrate as defined in any one of claims 1 to 3 comprising the steps of:
a) suspending 2-hydroxy-3-[5-(morpholin-4-ylmethyl)pyridin-2-yl]1*H*-indole-5-carbonitrile citrate of any form, or a mixture of any form in water;
b) heating the slurry to about 85°C until a solution is obtained;
c) crystallizing the obtained solution by cooling to about 45°C over about 30 min. and then further cooling down to about 5°C over about 20 hrs;
d) isolating the crystallized compound obtained, by filtering followed by washing with ethanol and drying under vacuum at about 50°C.

5. A substantially crystalline form of compound 2-hydroxy-3-[5-(morpholin-4-ylmethyl)pyridin-2-yl]1*H*-indole-5-carbonitrile citrate anhydrate as defined in any one of claims 1 to 3 prepared according to the process of claim 4.

6. A compound 2-hydroxy-3-[5-(morpholin-4-ylmethyl)pyridin-2-yl]1*H*-indole-5-carbonitrile citrate monohydrate in substantially crystalline form, which is a Form E **characterized by** the X-ray powder diffraction d-value **15.51** Å.

7. A compound 2-hydroxy-3-[5-(morpholin-4-ylmethyl)pyridin-2-yl]1*H*-indole-5-carbonitrile citrate monohydrate in substantially crystalline form, which is a Form E **characterized by** the X-ray powder diffraction d-values **15.51, 11.90, 5.17** Å.

8. A compound 2-hydroxy-3-[5-(morpholin-4-ylmethyl)pyridin-2-yl]1*H*-indole-5-carbonitrile citrate monohydrate in substantially crystalline form, which is a Form E **characterized by** the X-ray powder diffraction d-values and relative intensity **15.51, 11.90, 5.96, 5.71, 5.17, 3.61** Å.

9. A process for the preparation of a substantially crystalline form of 2-hydroxy-3-[5-(morpholin-4-ylmethyl)pyridin-2-yl]1*H*-indole-5-carbonitrile citrate monohydrate as defined in any one of claims 6 to 8 comprising the steps of:
a) suspending 2-hydroxy-3-[5-(morpholin-4-ylmethyl)pyridin-2-yl]1*H*-indole-5-carbonitrile citrate of any form, or a mixture of any form in water;
b heating the slurry to about 95°C until a solution is obtained;
c) cooling the obtained solution to about 75°C over about 90 min. and then adding ethanol in the ratio of about between 0% and 40% (v/v) to the water added;
d) crystallizing the obtained solution in step c) by further cooling to about 15°C over about 8 hrs and then stirring overnight;
e) isolating the crystallized compound obtained, by filtering followed by washing with acetone/water and pulled dry.

10. A substantially crystalline form of compound 2-hydroxy-3-[5-(morpholin-4-ylmethyl)pyridin-2-yl]1*H*-indole-5-carbonitrile citrate monohydrate as defined in any one of claims 6 to 8 prepared according to the process of claim 9.

11. A pharmaceutical formulation comprising as active ingredient a therapeutically effective amount of the compound according to any one of claims 1 to 3, 5 to 8 and 10 optionally in association with diluents or inert carriers.

12. A compound as defined in any one of claims 1 to 3, 5 to 8 and 10 for use in therapy.

13. Compound as defined in any one of claims 1 to 3, 5 to 8 and 10 for use in the prevention and/or treatment of conditions associated with glycogen synthase kinase-3, cognitive disorders, dementia, Cognitive Deficit in Schizophrenia (CDS), Mild Cognitive Impairment (MCI), Age-Associated Memory Impairment (AAMI), Age-Related Cognitive Decline (ARCD) or Cognitive Impairement No Dementia (CIND), dementia associated with neurofibrillar tangle pathologies, Frontotemporal dementia (FTD), Frontotemporal dementia Parkinson's Type (FTDP), progressive supranuclear palsy (PSP), Pick's Disease, Niemann-Pick's Disease, corticobasal degeneration, traumatic brain injury (TBI), dementia pugilistica, Alzheimer's Disease (AD), Down's syndrome, vascular dementia, Parkinson's Disease (PD), postencephelatic parkinsonism, dementia with Lewy bodies, HIV dementia, Huntington's Disease, amyotrophic lateral sclerosis (ALS), motor neuron diseases (MND, Creuztfeld-Jacob's disease, prion diseases, attention deficit disorder (ADD), attention deficit hyperactivity disorder (ADHD), affective disorders, Bipolar Disorder including acute mania, bipolar depression, bipolar maintenance, major depressive disorders (MDD) including depression, major depression, mood stabilization, schizoaffective disorders including schizophrenia, dysthymia, Type I diabetes, Type II diabetes, diabetic neuropathy, alopecia, inflammatory diseases, bone related disorders or conditions, including osteoporosis; increasing bone formation including increasing cancellous bone formation and/or new bone formation; increasing bone mineral density, reducing the incidence of fracture and enhancing fracture healing.

## Patentansprüche

1. Verbindung 2-Hydroxy-3-[5-(morpholin-4-ylmethyl)pyridin-2-yl]-1*H*-indol-5-carbonsäurenitrilcitrat-anhydrat in im Wesentlichen kristalliner Form, bei der es sich um eine Form D handelt, **gekennzeichnet durch** den Röntgenpulverdiffraktions-d-Wert 14,56 Å.

2. Verbindung 2-Hydroxy-3-[5-(morpholin-4-ylmethyl)pyridin-2-yl]-1*H*-indol-5-carbonsäurenitrilcitrat-anhydrat in im Wesentlichen kristalliner Form, bei der es sich um eine Form D handelt, **gekennzeichnet durch** die Röntgenpulverdiffraktions-d-Werte 14,56, 11,36 und 5,57 Å.

3. Verbindung 2-Hydroxy-3-[5-(morpholin-4-ylmethyl)pyridin-2-yl]-1*H*-indol-5-carbonsäurenitrilcitrat-anhydrat in im Wesentlichen kristalliner Form, bei der es sich um eine Form D handelt, **gekennzeichnet durch** die Röntgenpulverdiffraktions-d-Werte und relative Intensität 14,56, 11,36, 9,11, 5,57, 5,11, 4,29 und 3,52 Å.

4. Verfahren zur Herstellung einer im Wesentlichen kristallinen Form von 2-Hydroxy-3-[5-(morpholin-4-ylmethyl)pyridin-2-yl]-1*H*-indol-5-carbonsäurenitrilcitrat-anhydrat nach einem der Ansprüche 1 bis 3, bei dem man:
a) 2-Hydroxy-3-[5-(morpholin-4-ylmethyl)pyridin-2-yl]-1*H*-indol-5-carbonsäurenitrilcitrat in einer beliebigen Form oder als Mischung einer beliebigen Form in Wasser suspendiert;
b) die Suspension auf etwa 85°C erhitzt, bis sich eine Lösung gebildet hat;
c) die erhaltene Lösung durch Abkühlen auf etwa 45°C im Verlauf von etwa 30 min und dann weiteres Abkühlen auf etwa 5°C im Verlauf von etwa 20 Stunden kristallisiert;
d) die erhaltene kristallisierte Verbindung durch Filtrieren, anschließendes Waschen mit Ethanol und Trocknen im Vakuum bei etwa 50°C isoliert.

5. Im Wesentlichen kristalline Form der Verbindung 2-Hydroxy-3-[5-(morpholin-4-ylmethyl)pyridin-2-yl]-1*H-*indol-5-carbonsäurenitrilcitrat-anhydrat nach einem der Ansprüche 1 bis 3, hergestellt nach dem Verfahren von Anspruch 4.

6. Verbindung 2-Hydroxy-3-[5-(morpholin-4-ylmethyl)pyridin-2-yl]-1*H*-indol-5-carbonsäurenitrilcitrat-monohydrat in im Wesentlichen kristalliner Form, bei der es sich um eine Form E handelt, **gekennzeichnet durch** den Röntgenpulverdiffraktions-d-Wert 15,51 Å.

7. Verbindung 2-Hydroxy-3-[5-(morpholin-4-ylmethyl)pyridin-2-yl]-1*H*-indol-5-carbonsäurenitrilcitrat-monohydrat in im Wesentlichen kristalliner Form, bei der es sich um eine Form E handelt, **gekennzeichnet durch** die Röntgenpulverdiffraktions-d-Werte 15,51, 11,90 und 5,17 Å.

8. Verbindung 2-Hydroxy-3-[5-(morpholin-4-ylmethyl)pyridin-2-yl]-1*H*-indol-5-carbonsäurenitrilcitrat-monohydrat in im Wesentlichen kristalliner Form, bei der es sich um eine Form E handelt, **gekennzeichnet durch** die Röntgenpulverdiffraktions-d-Werte und relative Intensität 15,51, 11,90, 5,96, 5,71, 5,17 und 3,61 Å.

9. Verfahren zur Herstellung einer im Wesentlichen kristallinen Form von 2-Hydroxy-3-[5-(morpholin-4-ylmethyl)pyridin-2-yl]-1*H*-indol-5-carbonsäurenitrilcitrat-monohydrat nach einem der Ansprüche 6 bis 8, bei dem man:
a) 2-Hydroxy-3-[5-(morpholin-4-ylmethyl)pyridin-2-yl]-1*H*-indol-5-carbonsäurenitrilcitrat in einer beliebigen Form oder als Mischung einer beliebigen Form in Wasser suspendiert;
b) die Suspension auf etwa 95°C erhitzt, bis sich eine Lösung gebildet hat;
c) die erhaltene Lösung im Verlauf von etwa 90 min auf etwa 75°C abkühlt und dann in einem Verhältnis von etwa 0% bis 40% (v/v) zum zugesetzten Wasser mit Ethanol versetzt;
d) die in Schritt c) erhaltene Lösung durch weiteres Abkühlen auf etwa 15°C im Verlauf von etwa 8 Stunden und anschließendes Rühren über Nacht kristallisiert;
e) die erhaltene kristallisierte Verbindung durch Filtrieren, anschließendes Waschen mit Aceton/Wasser und Absaugen bis zur Trockne isoliert.

10. Im Wesentlichen kristalline Form der Verbindung 2-Hydroxy-3-[5-(morpholin-4-ylmethyl)pyridin-2-yl]-1*H-*indol-5-carbonsäurenitrilcitrat-monohydrat nach einem der Ansprüche 6 bis 8, hergestellt nach dem Verfahren von Anspruch 9.

11. Pharmazeutische Formulierung, enthaltend, als Wirkstoff, eine therapeutisch wirksame Menge der Verbindung nach einem der Ansprüche 1 bis 3, 5 bis 8 und 10, gegebenenfalls zusammen mit Verdünnungsmitteln oder inerten Trägern.

12. Verbindungen nach einem der Ansprüche 1 bis 3, 5 bis 8 und 10 zur Verwendung in der Therapie.

13. Verbindungen nach einem der Ansprüche 1 bis 3, 5 bis 8 und 10 zur Verwendung bei der Prävention und/oder Behandlung von mit Glykogensynthasekinase-3 assoziierten Leiden, kognitiven Störungen, Demenz, kognitivem Defizit bei Schizophrenie (Cognitive Deficit in Schizophrenia, CDS), milden kognitiven Störungen (Mild Cognitive Impairment, MCI), altersbedingten Gedächtnisstörungen (Age-Associated Memory Impairment, AAMI), altersbedingtem Nachlassen der kognitiven Fähigkeiten (Age-Related Cognitive Decline, ARCD) oder kognitiven Störungen ohne Demenz (Cognitive Impairment No Dementia, CIND), mit neurofibrillären Bündeln in Zusammenhang stehenden Pathologien assoziierte Demenz, frontotemporale Demenz (FTD), frontotemporale Demenz vom Parkinson-Typ (FTDP), progressive supranukleäre Pickparese (Progressive Supranuclear Palsy, PSP), Pick-Krankheit, Niemann-Pick-Krankheit, kortikobasale Degeneration, traumatische Gehirnverletzung (Traumatic Brain Injury, TBI), Dementia pugilistica, Alzheimer-Krankheit (Alzheimer's Disease, AD), Down-Syndrom, vaskulärer Demenz, Parkinson-Krankheit (Parkinson's Disease, PD), post-enzephalitischem Parkinsonismus, Demenz mit Lewis-Körpern, HIV-Demenz, Huntington-Krankheit, amyotropher Lateralsklerose (ALS), Motoneuron-Krankheiten (Motor Neuron Diseases, MND), Creutzfeld-Jacob-Krankheit, Prionenkrankheiten, Aufmerksamkeitsdefizitstörung (Attention Deficit Disorder, ADD), Aufmerksamkeitsdefizit-/Hyperaktivitätsstörung (Attention Deficit Hyperactivity Disorder, ADHD), affektiven Störungen, bipolaren Störungen einschließlich akuter Manie, bipolarer Depression, bipolarer Erhaltung, schweren depressiven Störungen (Major Depressive Disorders, MDD) einschließlich Depression, schwerer Depression, Stimmungsstabilisation, schizoaffektiven Störungen einschließlich Schizophrenie, Dysthymie, Typ-I-Diabetes, Typ-II-Diabetes, diabetischer Neuropathie, Alopecie, entzündlichen Krankheiten, mit den Knochen in Zusammenhang stehenden Erkrankungen bzw. Leiden einschließlich Osteoporose; bei der vermehrten Bildung von Knochen einschließlich der vermehrten Bildung von Substantia spongiosa und/oder Bildung von neuem Knochen; der Erhöhung der Knochenmineraldichte, der Verminderung der Häufigkeit von Brüchen und der verbesserten Heilung von Brüchen.

## Revendications

1. Composé citrate de 2-hydroxy-3-[5-(morpholin-4-ylméthyl)pyridin-2-yl]-1*H*-indole-5-carbonitrile anhydre sous forme pratiquement cristalline, qui est une forme D **caractérisée par** la valeur de d déterminée par diffraction des rayons X sur poudre de 14,56 Å.

2. Composé citrate de 2-hydroxy-3-[5-(morpholin-4-ylméthyl)pyridin-2-yl]-1*H*-indole-5-carbonitrile anhydre sous forme pratiquement cristalline, qui est une forme D **caractérisée par** les valeurs de d déterminées par diffraction des rayons X sur poudre de 14,56, 11,36 et 5,57 Å.

3. Composé citrate de 2-hydroxy-3-[5-(morpholin-4-ylméthyl)pyridin-2-yl]-1*H*-indole-5-carbonitrile anhydre sous forme pratiquement cristalline, qui est une forme D **caractérisée par** les valeurs de d et l'intensité relative déterminées par diffraction des rayons X sur poudre de 14,56, 11,36, 9,11, 5,57, 5,11, 4,29 et 3,52 Å.

4. Procédé pour la préparation d'une forme pratiquement cristalline du citrate de 2-hydroxy-3-[5-(morpholin-4-ylméthyl)pyridin-2-yl]-1*H*-indole-5-carbonitrile anhydre selon l'une quelconque des revendications 1 à 3 comprenant les étapes consistant à :
a) mettre en suspension du citrate de 2-hydroxy-3-[5-(morpholin-4-ylméthyl)pyridin-2-yl]-1*H*-indole-5-carbonitrile sous n'importe quelle forme, ou un mélange de n'importe quelles formes, dans de l'eau ;
b) chauffer la suspension épaisse à environ 85 °C jusqu'à ce qu'une solution soit obtenue ;
c) faire cristalliser la solution obtenue par refroidissement à environ 45 °C sur une durée d'environ 30 min et ensuite refroidir encore à environ 5 °C sur une durée d'environ 20 h ;
d) isoler le composé cristallisé obtenu, par filtration suivie par le lavage avec de l'éthanol et le séchage sous vide à environ 50 °C.

5. Forme pratiquement cristalline du composé citrate de 2-hydroxy-3-[5-(morpholin-4-ylméthyl)pyridin-2-yl]-1*H*-indole-5-carbonitrile anhydre selon l'une quelconque des revendications 1 à 3 préparée selon le procédé selon la revendication 4.

6. Composé citrate de 2-hydroxy-3-[5-(morpholin-4-ylméthyl)pyridin-2-yl]-1*H*-indole-5-carbonitrile monohydraté sous forme pratiquement cristalline, qui est une forme E **caractérisée par** la valeur de d déterminée par diffraction des rayons X sur poudre de 15,51 Å.

7. Composé citrate de 2-hydroxy-3-[5-(morpholin-4-ylméthyl)pyridin-2-yl]-1*H*-indole-5-carbonitrile monohydraté sous forme pratiquement cristalline, qui est une forme E **caractérisée par** les valeurs de d déterminées par diffraction des rayons X sur poudre de 15,51, 11,90 et 5,17 Å.

8. Composé citrate de 2-hydroxy-3-[5-(morpholin-4-ylméthyl)pyridin-2-yl]-1*H*-indole-5-carbonitrile monohydraté sous forme pratiquement cristalline, qui est une forme E **caractérisée par** les valeurs de d et l'intensité relative déterminées par diffraction des rayons X sur poudre de 15,51, 11,90, 5,96, 5,71, 5,17 et 3,61 Å.

9. Procédé pour la préparation d'une forme pratiquement cristalline du citrate de 2-hydroxy-3-[5-(morpholin-4-ylméthyl)pyridin-2-yl]-1*H*-indole-5-carbonitrile monohydraté selon l'une quelconque des revendications 6 à 8 comprenant les étapes consistant à :
a) mettre en suspension du citrate de 2-hydroxy-3-[5-(morpholin-4-ylméthyl)pyridin-2-yl]-1*H*-indole-5-carbonitrile sous n'importe quelle forme, ou un mélange de n'importe quelles formes, dans de l'eau ;
b) chauffer la suspension épaisse à environ 95 °C jusqu'à ce qu'une solution soit obtenue ;
c) refroidir la solution obtenue à environ 75 °C sur une durée d'environ 90 min et ensuite ajouter de l'éthanol en proportion comprise entre environ 0 % et 40 % (v/v) à l'eau ajoutée ;
d) faire cristalliser la solution obtenue dans l'étape c) par refroidissement additionnel à environ 15 °C sur une durée d'environ 8 h et ensuite agiter pendant la nuit ;
e) isoler le composé cristallisé obtenu, par filtration suivie par le lavage avec de l'acétone/eau et séchage par aspiration.

10. Forme pratiquement cristalline du composé citrate de 2-hydroxy-3-[5-(morpholin-4-ylméthyl)pyridin-2-yl]-1*H*-indole-5-carbonitrile monohydraté selon l'une quelconque des revendications 6 à 8 préparée selon le procédé selon la revendication 9.

11. Formulation pharmaceutique comprenant comme principe actif une quantité thérapeutiquement efficace du composé selon l'une quelconque des revendications 1 à 3, 5 à 8 et 10 facultativement en association avec des diluants ou véhicules inertes.

12. Composé selon l'une quelconque des revendications 1 à 3, 5 à 8 et 10 destiné à être utilisé en thérapie.

13. Composé selon l'une quelconque des revendications 1 à 3, 5 à 8 et 10 destiné à être utilisé dans la prévention et/ou le traitement d'affections associées à la glycogène synthase kinase 3, de troubles cognitifs, de la démence, du déficit cognitif en schizophrénie (CDS), d'un trouble cognitif léger (MCI), d'un trouble de la mémoire associé à l'âge (AAMI), d'un déclin cognitif lié à l'âge (ARCD) ou d'un trouble cognitif sans démence (CIND), de la démence associée à des pathologies de dégénérescence neurofibrillaire, de la démence frontotemporale (FTD), de la démence frontotemporale de type Parkinson (FTDP), de la maladie de Steele-Richardson (PSP), de la maladie de Pick, de la maladie de Niemann-Pick, de la dégénérescence corticobasale, d'une lésion traumatique du cerveau (TBI), de la démence pugilistique, de la maladie d'Alzheimer (AD), de la trisomie 21, de la démence vasculaire, de la maladie de Parkinson (PD), du syndrome parkinsonien post-encéphalique, de la démence à corps de Lewis, de la démence à VIH, de la maladie de Huntington, de la sclérose latérale amyotrophique (ALS), de maladies des neurones moteurs (MND), de la maladie de Creutzfeld-Jacob, de maladies à prion, du trouble déficitaire de l'attention (ADD), du trouble d'hyperactivité avec déficit de l'attention (ADHD), de troubles affectifs, d'un trouble bipolaire dont une manie aiguë, de la dépression bipolaire, d'entretien de troubles bipolaires, de troubles dépressifs majeurs (MDD) dont la dépression, de la dépression majeure, de stabilisation de l'humeur, de troubles schizo-affectifs dont la schizophrénie, de la dysthymie, du diabète de type I, du diabète de type II, d'une neuropathie diabétique, de l'alopécie, de maladies inflammatoires, de troubles ou affections liés aux os, dont l'ostéoporose ; l'augmentation de la formation osseuse dont l'augmentation de la formation d'os spongieux et/ou la formation de nouveaux os ; l'augmentation de la densité minérale osseuse, la réduction de la fréquence de fractures et l'amélioration de la cicatrisation de fractures.
